(19) Europäisches Patentamt
European Patent Office
Office européen des brevets

(11) Numéro de publication: **0 502 784 B1**

(12) # FASCICULE DE BREVET EUROPEEN

(45) Date de publication de fascicule du brevet: **21.06.95**

(51) Int. Cl.$^6$: **A61K 7/13**

(21) Numéro de dépôt: **92400558.0**

(22) Date de dépôt: **04.03.92**

(54) **Procédé de teinture des fibres kératiniques associant l'isatine ou ses dérivés à une aniline tri-, tétra- ou pentasubstituée ou à une bis-phénylalkylènediamine, et agents de teinture.**

(30) Priorité: **05.03.91 FR 9102615**

(43) Date de publication de la demande:
**09.09.92 Bulletin 92/37**

(45) Mention de la délivrance du brevet:
**21.06.95 Bulletin 95/25**

(84) Etats contractants désignés:
**AT BE CH DE DK ES FR GB GR IT LI NL PT SE**

(56) Documents cités:
**EP-A- 0 359 465**
**FR-A- 2 626 771**
**US-A- 4 921 503**

(73) Titulaire: **L'OREAL**
**14, rue Royale**
**F-75008 Paris (FR)**

(72) Inventeur: **Lang, Gérard**
**44, avenue Lacour**
**F-95210 Saint-Gratien (FR)**
Inventeur: **Cotteret, Jean**
**15 Allée des Meuniers**
**F-78480 Verneuil-sur-Seine (FR)**

(74) Mandataire: **Casalonga, Axel et al**
**BUREAU D.A. CASALONGA - JOSSE**
**Morassistrasse 8**
**D-80469 München (DE)**

**EP 0 502 784 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de teinture des fibres kératiniques, en particulier des cheveux humains, associant l'isatine ou l'un de ses dérivés à une diamine aromatique ou un aminophénol di-, tri- ou tétrasubstitué ou à une bis-phénylalkylènediamine, ainsi que les agents de teinture mis en oeuvre.

En coloration directe des cheveux, c'est-à-dire dans un procédé de teinture ne mettant pas en oeuvre un processus de développement des colorants par voie oxydative, on a déjà proposé d'utiliser la 2,3-indolinedione encore appelée isatine comme colorant jaune de base dans le brevet français n° 2.588.473.

La demande européenne n° 0359.465 a proposé ensuite un procédé de teinture directe utilisant l'isatine ou l'un de ses dérivés en association avec des dérivés d'aminobenzène disubstitués.

La demanderesse vient de découvrir, d'une manière surprenante, un nouveau procédé de teinture associant l'isatine ou ses dérivés à des colorants du type aniline tri-, tétra- ou pentasubstituée ou à des composés du type bis-phénylalkylènediamine, permettant d'obtenir une large gamme de nuances plus résistantes aux shampooings et à la transpiration que celles obtenues avec les procédés de teinture directe utilisant les dérivés aminés connus de l'art antérieur. Les colorations obtenues sont de plus stables à la lumière, aux intempéries et aux agents chimiques.

La présente invention a donc pour objet un procédé de teinture des fibres kératiniques, consistant à appliquer sur les fibres, l'isatine ou l'un de ses dérivés, et une diamine aromatique ou un aminophénol di-, tri- ou tétrasubstitué ou une bis-phénylalkylènediamine, soit simultanément sous forme d'un mélange extemporané, soit de façon successive.

Un objet de l'invention consiste également en un agent de teinture à deux composants.

D'autres objets apparaîtront à la lumière de la description.

Le procédé de teinture des fibres kératiniques, en particulier des cheveux humains, conforme à la présente invention, est caractérisé essentiellement par le fait qu'il comporte l'application sur lesdites fibres d'un composant (A) constitué d'une composition contenant dans un milieu approprié pour la teinture, au moins un composé de formule (I) suivante :

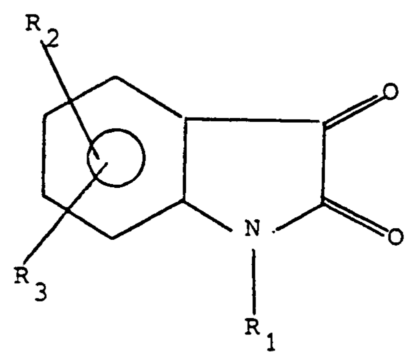

(I)

dans laquelle :

$R_1$ désigne un atome d'hydrogène, un radical alkyle en $C_1$-$C_6$, acétyle, benzoyle, phényle ou carboxyalkyle en $C_1$-$C_4$;

$R_2$ et $R_3$, indépendamment l'un de l'autre, désignent un atome d'hydrogène, un alkyle en $C_1$-$C_6$, un alcoxy en $C_1$-$C_4$, un hydroxyle, un amino, un atome d'halogène, un groupement nitro, un alkyl($C_1$-$C_6$)-phényle, phényle, alkyl($C_1$-$C_6$)amino, hydroxyalkyl($C_1$-$C_6$)amino, polyhydroxyalkyl($C_2$-$C_6$)amino;

et un composant (B) constitué d'une composition contenant dans un milieu approprié pour la teinture, au moins

(i) une diamine aromatique ou un aminophénol di-, tri- ou tétrasubstitué de formule (II) suivante :

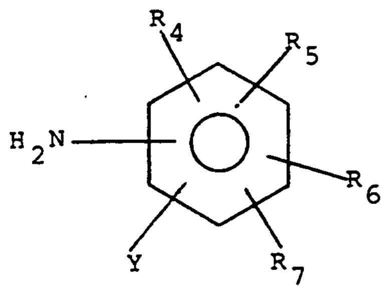

(II)

dans laquelle :

Y désigne OH ou $NR_8R_9$;

$R_8$ et $R_9$, identiques ou différents, désignent hydrogène, aminoéthyle, hydroxyéthyle ou alkyle en $C_1$-$C_4$;

$R_4$ à $R_7$, indépendamment ent l'un de l'autre, désignent un atome d'hydrogène, un alkyle en $C_1$-$C_4$, un chlore, un acétylamino, un alcoxy en $C_1$-$C_4$, ou un aryloxy;

au plus deux des groupements $R_4$ à $R_7$ désignant un atome d'hydrogène;

à l'exclusion du 2,5-diméthoxy 1,4-diaminobenzène;

ainsi que les sels cosmétiquement acceptables de ces composés; ou

(ii) une bis-phénylalkylènediamine, dite base double, de formule (IV) :

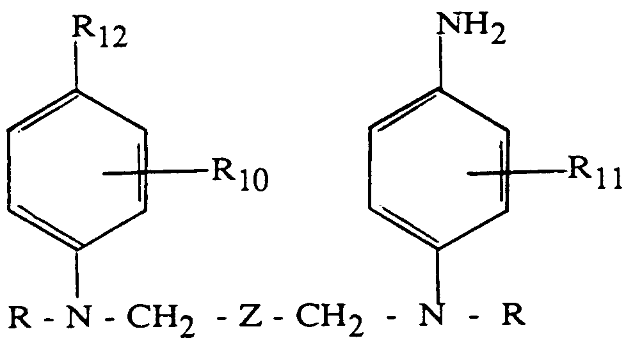

(IV)

dans laquelle :

$R_{12}$ représente un groupement hydroxyle ou $NHR_{13}$, où $R_{13}$ désigne un atome d'hydrogène ou un radical alkyle inférieur;

$R_{10}$ et $R_{11}$, identiques ou différents, représentent soit des atomes d'hydrogène, soit des atomes d'halogène, soit encore des groupements alkyle;

R représente un atome d'hydrogène, un groupe alkyle, hydroxyalkyle ou aminoalkyle, dont le reste amino peut être substitué;

Z représente un radical pris dans le groupe constitué par les radicaux suivants :

$-(CH_2)_n-$, $-(CH_2)_{n'}-O-(CH_2)_{n'}-$,

$-(CH_2)_{n'}-CHOH-(CH_2)_{n'}-$,

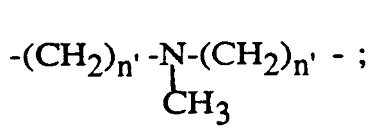

<u>n</u> étant un nombre entier compris entre 0 et 8 et <u>n'</u> un nombre entier compris entre 0 et 4, cette base pouvant se présenter sous forme de ses sels d'addition avec des acides.

A titre de radicaux alkyle en $C_1$-$C_4$, on peut citer les radicaux méthyle, éthyle, n-propyle, n-butyle, isopropyle, isobutyle et tert.-butyle.

Les radicaux alkyle en $C_1$-$C_6$ comportent, outre ceux cités ci-dessus, les radicaux pentyle (linéaires et ramifiés) et hexyle (linéaires et ramifiés).

A titre de radicaux alcoxy en $C_1$-$C_4$, on peut citer les radicaux méthoxy, éthoxy, propoxy et butoxy.

Les radicaux alcoxy en $C_1$-$C_6$ comportent, outre ceux cités ci-dessus, les radicaux pentyloxy et hexyloxy.

A titre de radicaux hydroxyalkyle, on peut citer hydroxyéthyle; les 2- ou 3-hydroxypropyle.

A titre de radicaux polyhydroxyalkyle, on peut citer les 2,3-dihydroxypropyle, 3,4-dihydroxybutyle.

Pour ce qui est des radicaux aryloxy, on peut citer phényloxy et benzyloxy.

Les sels cosmétiquement acceptables sont choisis parmi les chlorhydrates, bromhydrates et sulfates.

Dans la définition des bases dites doubles, les radicaux alkyle ou alcoxy désignent de préférence un groupement ayant 1 à 4 atomes de carbone et notamment méthyle, éthyle, propyle, méthoxy, éthoxy.

Le procédé selon l'invention peut être mis en oeuvre sans l'intervention d'un agent oxydant autre que l'air.

Le procédé de teinture décrit ci-dessus conduit à la formation d'une base de Schiff, soit lors du mélange de la composition (A) avec la composition (B), soit in situ dans la fibre kératinique lors d'une application séquentielle des compositions (A) et (B). Cette base de Schiff a pour formule :

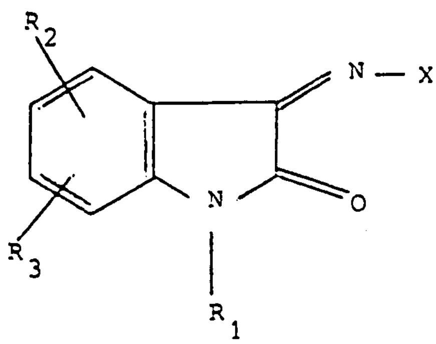

(III)

dans laquelle X désigne :

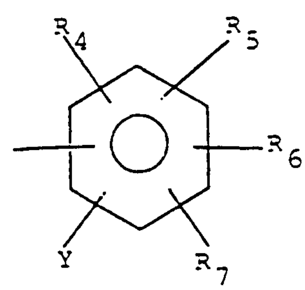

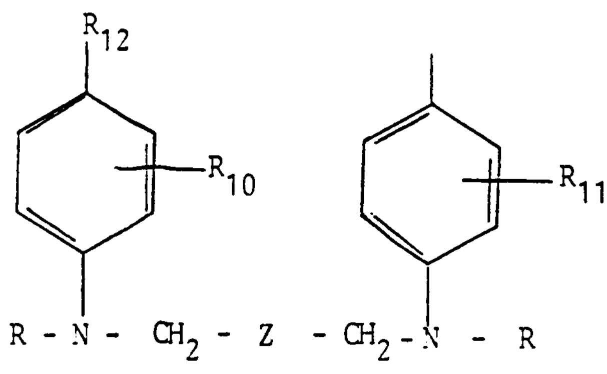

et Z, R, $R_{10}$ à $R_{12}$;

Y et $R_4$ à $R_7$ ayant les significations définies ci-dessus.

Parmi les composés de formule (I), on peut citer plus particulièrement l'isatine.

Les composés de formule (II) préférentiels sont choisis parmi :

le 2-méthoxy 3,5-diméthyl 1,4-diaminobenzène
le 2,6-diméthyl 4-bis-(β-hydroxyéthyl)amino 1-aminobenzène
le 5,6-diméthoxy 1,3-diaminobenzène
le 2,6-diméthyl 1,3-diaminobenzène
le 2,6-diméthoxy 1,3-diaminobenzène
le 2,6-diméthoxy 5-chloro 1,3-diaminobenzène
le 2,6-diméthoxy 3-β-hydroxyéthylamino 1-aminobenzène
le 2,4-diméthoxy 3-β-hydroxyéthylamino 1-aminobenzène
le 4,6-dibenzyloxy 1,3-diaminobenzène
le 3-métyl 6-méthoxy 1,2-diaminobenzène
le 3,5-diméthyl 4-aminophénol
le 2,5-diméthyl 4-aminophénol
le 2,3,5-triméthyl 4-aminophénol
le 2,3,5,6-tétraméthyl 4-aminophénol
le 4-chloro 5-acétylamino 2-aminophénol, et
le 4,6-diphényloxy 1,3-diaminobenzène.

En plus de ces composés préférés, les composés de formule (II) plus particulièrement préférés sont choisis parmi :

le 2,6-diméthyl 1,4-diaminobenzène
le 2,5-diméthyl 1,4-diaminobenzène
le 2,3-diméthyl 1,4-diaminobenzène
le 2-méthoxy 5-méthyl 1,4-diaminobenzène
le 2-méthoxy 5-méthyl 4-β-aminoéthylamino 1-aminobenzène
le 2-méthyl 5-chloro 4-β-aminoéthylamino 1-aminobenzène
le 4,6-diméthoxy 1,3-diaminobenzène
le 2,3-dimétyl 4-aminophénol
le 2,6-diméthyl 4-aminophénol, et
le 2,5-diméthyl 3-aminophénol.

Parmi les composés de formule (IV), on peut citer le N,N'bis-(β-hydroxyéthyl)N,N'-bis(4'-aminophényl) 1,3-diamino 2-propanol, la N,N'bis-(β-hydroxyéthyl)N,N'-bis(4'-aminophényl)éthylènediamine, la N,N'bis-(4-aminophényl)tétraméthylènediamine, la N,N'bis-(β-hydroxy éthyl)N,N'bis(4-aminophényl)-tétraméthylènediamine, la N,N'bis(éthyl) N,N'-bis(4'-amino 3'-méthylphényl)éthylènediamine.

Selon le procédé de la présente invention, les composés de formule (I) sont de préférence présents dans le composant (A) dans des proportions comprises entre 0,01 et 5% en poids et plus particulièrement entre 0,25 et 2% en poids par rapport au poids total du composant (A) ou des composants (A) + (B) et les composés de formule (II) ou (IV) sont présents dans le composant (B) dans des proportions comprises de préférence entre 0,01 et 5% en poids et en particulier entre 0,25 et 2% en poids par rapport au poids total du composant (B) ou des composants (A) + (B).

Les composants (A) et (B) utilisables conformément à l'invention, sont des compositions liquides, plus ou moins épaissies, aqueuses ou anhydres, des crèmes, des gels aqueux ou anhydres, des huiles ou des poudres à diluer avec un liquide au moment de l'emploi, encore appelées "cataplasmes".

Dans une première forme de réalisation de l'invention, le milieu cosmétique approprié pour la teinture est aqueux et a un pH pouvant varier entre 2 et 10, et de préférence entre 3 et 9,5, il est ajusté à la valeur désirée à l'aide d'agents alcalinisants ou d'agents acidifiants connus en eux-mêmes.

Ces compositions peuvent contenir des agents tensio-actifs anioniques, cationiques, non-ioniques, amphotères ou leurs mélanges. Ces tensio-actifs sont présents dans les compositions conformes à l'invention dans des proportions comprises entre 0,1 et 55% en poids, et de préférence entre 1 et 40% en poids par rapport au poids total de chaque composition.

Ces compositions aqueuses peuvent renfermer des solvants organiques parmi lesquels on peut mentionner à titre d'exemple, les alcanols inférieurs tels que l'éthanol ou l'isopropanol, les polyols tels que le glycérol, les glycols ou éthers de glycols comme l'éthylène glycol, le propylèneglycol, l'éther monobutylique de l'éthylène glycol, le monoéthyléther et le monométhyléther de diéthylène glycol ainsi que des produits analogues ou leurs mélanges.

Ces solvants sont de préférence utilisés dans des proportions allant de 1 à 60% en poids, et plus particulièrement de 3 à 30% en poids par rapport au poids total de chaque composition.

Ces compositions peuvent être épaissies avec des agents choisis parmi l'alginate de sodium, la gomme arabique, la gomme de guar ou de caroube, la gomme de xanthane, les scléroglucanes, les pectines, les dérivés de la cellulose et les polymères divers ayant une fonction épaississante tels que les dérivés d'acide acrylique. On peut également utiliser des agents épaississants minéraux tels que la bentonite.

Ces agents épaississants sont présents de préférence dans des proportions comprises entre 0,1 et 5% en poids et en particulier entre 0,5 et 3% en poids par rapport au poids total de chaque composition.

Ces compositions peuvent également contenir des polymères anioniques, non-ioniques, cationiques, amphotères ou leurs mélanges, en des proportions de 0,1 à 5% en poids par rapport au poids total de chaque composition.

Ces compositions peuvent bien entendu contenir tous autres adjuvants habituellement utilisés dans les compositions pour la teinture des cheveux, tels que les agents de pénétration, les agents séquestrants, les agents antioxydants, des tampons, des parfums, des colorants, etc...

Une forme préférée de l'invention consiste à utiliser un milieu anhydre tel que décrit dans le brevet français n° 2.526.031.

On entend par milieu anhydre un milieu ne contenant pas plus de 1% d'eau.

Le milieu anhydre est constitué conformément à cette variante de l'invention, par un mélange d'au moins un solvant anhydre et d'un ou plusieurs agents tensio-actifs anhydres, de telle sorte que ces compositions contiennent au moins 15% de solvant et au moins 20% d'agent tensio-actif.

Les solvants utilisés sont des solvants cosmétiquement acceptables choisis parmi les monoalcools saturés en $C_2$-$C_{20}$ tels que l'éthanol, l'isopropanol, l'alcool cétylique ou l'octyldodécanol; les polyols tels que les alcoylèneglycols comme l'éthylèneglycol, le propylèneglycol, le glycérol, le diéthylèneglycol; les éthers de glycols tels que les mono-, di- et triéthylèneglycolmonoalcoyléthers comme par exemple l'éthylèneglycolmonoéthyléther, l'éthylèneglycolmonobutyléther, le diéthylèneglycolmonoéthyléther; des esters comme par exemple l'acétate de monométhyléther de l'éthylèneglycol, l'acétate de monoéthyléther de l'éthylèneglycol; les esters d'acides gras et d'alcools inférieurs saturés comme le myristate ou le palmitate d'isopropyle.

Les compositions particulièrement préférées contiennent un solvant choisi parmi l'éthanol, l'alcool cétylique, le propylèneglycol, l'éthylèneglycolmonoéthyléther ou l'éthylèneglycolmonobutyléther.

Les agents tensio-actifs utilisés dans cette forme de réalisation sont choisis parmi les agents tensio-actifs anhydres de type anionique, non-ionique, cationique, amphotère ou leurs mélanges. On peut citer plus particulièrement les alcools gras polyoxyéthylénés, les alkylphénols ou naphtols polyoxyéthylénés, les halogénures de monoalkyltriméthylammonium, les halogénures de dialkyldiméthylammonium, les savons, les alcools gras polyglycérolés. Les agents tensio-actifs préférés sont les agents tensio-actifs non-ioniques.

Ces compositions peuvent contenir un agent alcalin ou acidifiant anhydre tel que par exemple l'acide citrique, l'acide ascorbique, l'acide acétique, l'acide lactique et des alcanolamines telles que, de préférence, celles qui sont totalement substituées sur le groupement amine comme le diméthylaminoéthanol.

En-dehors des composés décrits ci-dessus, les compositions anhydres conformes à l'invention peuvent contenir de nombreux additifs utilisables en cosmétique à la seule condition qu'ils contiennent moins de 1% d'eau. Parmi ces additifs, on peut citer les parfums, les agents épaississants, les agents traitants, les agents antioxydants, les huiles végétales ou minérales, les agents conservateurs et les sels organiques.

Ces compositions peuvent être appliquées telles quelles sur les cheveux mouillés ou être diluées tout juste avant l'emploi. Dans ce dernier cas, au moment de la teinture, les compositions selon l'invention sont diluées avec une solution aqueuse, de telle sorte que le rapport entre la composition conforme à l'invention et la solution aqueuse soit compris entre 0,25 et 2. La solution aqueuse peut être constituée par de l'eau pure, mais également par tout autre liquide aqueux complexe plus ou moins épaissi tel que par exemple un support habituellement utilisé dans les compositions tinctoriales pour cheveux.

Dans ce cas, les composants du milieu cosmétique peuvent être tous types d'ingrédients cosmétiquement acceptables, anhydres ou non, habituellement utilisés dans ce type de composition et décrits de façon générale ci-dessus.

Une autre forme d'utilisation des composants (A) et/ou (B) conformes à l'invention, est constituée par l'utilisation sous forme de cataplasmes, c'est-à-dire sous forme de poudre à diluer avec un liquide au moment de l'emploi.

Dans cette forme de réalisation, les colorants sont préparés sous forme de poudre stable au stockage et introduits dans un milieu solide pouvant être constitué de poudres, de farines, de substances amylacées ou mucilagineuses que l'on dilue au moment de l'emploi avec un liquide adéquat de façon à former un mélange ayant une consistance appropriée pour être appliqué sur tête.

Les poudres ou farines utilisées dans ce type de composition sont constituées généralement par des substances insolubles telles que des silices, des argiles, des végétaux pulvérisés après extraction de leurs principes actifs par solvant.

Le liquide peut être constitué par de l'eau ou des mélanges d'eau et de solvants cosmétiquement acceptables tels que des alcools ou des glycols ou encore par des huiles.

Le milieu liquide est additionné à la poudre dans des proportions telles qu'après mélange, on obtienne une pâte ayant une viscosité comprise entre 0,3 et 5 Pa.s.

Un objet de l'invention est constitué par un agent de teinture pour les fibres kératiniques, en particulier des cheveux humains, caractérisé par le fait qu'il est constitué par les composants (A) et (B) stockés sous forme séparée, tels que définis ci-dessus.

Les composants (A) et (B) sont destinés, soit à être mélangés tout juste avant emploi, soit à être appliqués de façon successive sur les fibres à traiter.

Selon une forme de réalisation, on peut conditionner les différents composants (A) et (B) dans un dispositif à plusieurs compartiments encore appelé "kit de teinture" comportant tous les composants destinés à être appliqués pour une même teinture sur les fibres kératiniques, en particulier les cheveux, en applications successives avec ou sans prémélange.

De tels dispositifs peuvent comporter un premier compartiment contenant le composant (A) renfermant l'isatine ou ses dérivés de formule (I) et un second compartiment comportant le composant (B) renfermant l'aniline tri-, tétra- ou pentasubstituée de formule (II) ou la bis-phénylalkylènediamine de formule (IV).

Une autre variante peut également consister à stocker le composant (A) ou le composant (B) dans un milieu solvant anhydre et à prévoir un troisième compartiment contenant un milieu aqueux approprié pour la teinture et cosmétiquement acceptable. Dans ce cas, on mélange tout juste avant l'emploi le contenu du troisième compartiment dans l'un ou l'autre ou les deux compartiments contenant les composants anhydres (A) et (B) ou alors on mélange avant emploi les trois compartiments.

Selon une variante, le procédé de l'invention consiste à mélanger juste avant l'emploi le composant (A) au composant (B), la composition résultante étant appliquée sur les cheveux pendant 5 à 40 minutes et de préférence 20 à 30 minutes. Les cheveux sont alors rincés, lavés au shampooing, rincés à nouveau puis séchés.

Selon une autre variante, le procédé de l'invention consiste à appliquer sur les cheveux au moins un composant (A) et, avant ou après le composant (A), le composant (B), tels que définis ci-dessus; à laisser poser chacun d'entre eux pendant 5 à 40 minutes, de préférence 20 à 30 minutes, en rinçant éventuellement à l'eau entre les deux étapes. Les cheveux sont alors rincés, lavés au shampooing, rincés à nouveau puis séchés.

Les exemples suivants sont destinés à illustrer l'invention sans pour autant présenter un caractère limitatif.

EXEMPLES 1 à 15

On procède à la teinture des cheveux en appliquant sur des cheveux naturels gris à 90% de blancs, 20 g des compositions.

Les compositions sont préparées juste avant l'emploi.

On laisse agir la composition pendant 20 minutes, puis on rince les cheveux, on effectue un shampooing puis on rince à nouveau. Après séchage, les cheveux sont teints dans la nuance précisée au bas du tableau I ci-après.

T A B L E A U   I

| en g MA | 1 | 2 | 3 | 4 | 5 | 6 | 7 | 8 | 9 | 10 |
|---|---|---|---|---|---|---|---|---|---|---|
| Isatine | 1 | 1 | 1 | 1 | 1 | 1 | 1 | 1 | 1 | 1 |
| 2-méthoxy 4-β-aminoéthylamino 5-méthyl 1-aminobenzène, 3 HCl | 1 | | | | | | | | | |
| 2-méthyl 4-β-aminoéthylamino 5-chloro 1-aminobenzène, 3 HCl | | 1 | | | | | | | | |
| 4,6-diméthoxy 1,3-diaminobenzène, 2 HCl | | | 1 | | | | | | | |
| 2,3-diméthyl paraaminophénol | | | | 1 | | | | | | |
| 2,6-diméthyl paraaminophénol | | | | | 1 | | | | | |
| 2,5-diméthyl métaaminophénol | | | | | | 1 | | | | |
| 2,3,5,6-tétraméthyl paraaminophénol | | | | | | | 1 | | | |
| 4-chloro 5-acétylamino orthoaminophénol | | | | | | | | 1 | | |
| 2,5-diméthyl 1,4-diaminobenzène | | | | | | | | | 1 | |
| 2,3-diméthyl 1,4-diaminobenzène | | | | | | | | | | 1 |
| | | | | | | | | | | |
| Alcool éthylique | 30 | 30 | 30 | 30 | 30 | 30 | 30 | 30 | 30 | 30 |
| Triéthanolamine qs pH | 7,9 | 8 | 8 | | 8 | | 8 | 8 | 8 | 8 |
| pH spontané | | | | 8,1 | | 8 | | | | |
| Eau qsp | 100 | 100 | 100 | 100 | 100 | 100 | 100 | 100 | 100 | 100 |
| Nuances obtenues | violine | cuivré doré | blond très clair | cuivré | cuivré | doré mat | doré cuivré | blond très clair naturel lég. cendré | doré cuivré | doré cuivré |

TABLEAU I (suite)

| en g MA | 11 | 12 | 13 | 14 | 15 |
|---|---|---|---|---|---|
| Isatine | 1 | | 1 | 1 | 0,5 |
| 6-bromoisatine | | 1 | | | |
| 2,6-diméthyl 1,4-diaminobenzène | 1 | 1 | | | |
| 2-méthoxy 5-méthyl 1,4-diaminobenzène | | | 1 | | 0,5 |
| 2-méthoxy 3,5-diméthyl 1,4-diaminobenzène | | | | 1 | |
| Alcool éthylique | 30 | 30 | 30 | 30 | 30 |
| Lauryléthersulfate de sodium oxyéthyléné à 2 moles d'oxyde d'éthylène à 28% MA | | | | | 8,4 |
| Triéthanolamine qs pH | 8 | 8 | 8 | 8 | 8 |
| Eau qsp | 100 | 100 | 100 | 100 | 100 |
| Nuances obtenues | cuivré lég. rouge | blond clair beige nacré | acajou cuivré | doré mat | blond nacré cuivré |

EXEMPLE 16

On procède de la même façon qu'à l'exemple 14 en remplaçant le 2-méthoxy 5-méthyl 1,4-diaminobenzène par la N,N'-bis-(β-hydroxyéthyl)N,N'-bis-(4'-aminophényl) 1,3-diamino 2-propanol, 3 HCl.

Après séchage, les cheveux sont teints dans une nuance acajou cuivré.

EXEMPLE 17

Au moment de l'emploi, on mélange la composition (A) avec 2 fois son poids en eau et la composition (B) avec 1,5 fois son poids en eau. On applique 20 g de la composition (A) (pH8,6) sur 3 g de cheveux naturels gris à 90% de blancs.

On laisse agir la composition pendant 15 minutes, on rince puis on applique 20 g de la composition (B) (pH9,2) pendant 15 minutes, on rince les cheveux, on effectue un shampooing puis on rince à nouveau. Après séchage, les cheveux sont teints dans la nuance précisée au bas du tableau II ci-après.

TABLEAU II

| en g MA | 17 | |
|---|---|---|
| | Comp. A | Comp. B |
| Isatine | 4 | |
| 2,6-diméthyl 1,4-diaminobenzène | | 1 |
| Gomme de caroube vendue sous la dénomination Vidogum L 175 par la Société Sanofi Bio Industrie | 3 | |
| Carbonate de calcium | 8 | |
| Poudre de résidus d'épuisement de saponaire de granulométrie inférieure à 90 microns | 35 | |
| Lait écrémé en poudre qsp | 100 | |
| Alcool éthylique | | 28,5 |
| N,N-diméthylamino 2-éthanol | | 1 |
| Nonylphénol oxyéthylèné à 9 moles d'oxyde d'éthylène qsp | | 100 |
| Nuance obtenue | | blond clair beige |

## Revendications

**1.** Procédé de teinture des fibres kératiniques, en particulier des cheveux humains, caractérisé par le fait que l'on applique sur lesdites fibres un composant (A) constitué d'une composition renfermant dans un milieu approprié pour la teinture, au moins un composé de formule (I) :

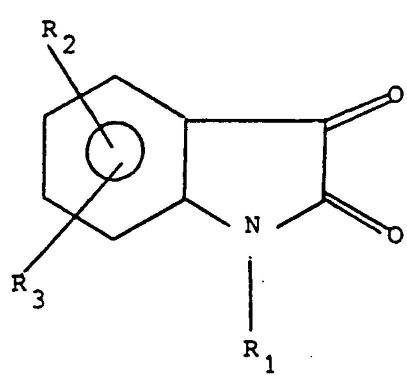

(I)

dans laquelle :

$R_1$ désigne un atome d'hydrogène, un radical alkyle en $C_1$-$C_6$, acétyle, benzoyle, phényle ou carboxyalkyle en $C_1$-$C_4$;

$R_2$ et $R_3$, indépendamment l'un de l'autre, désignent un atome d'hydrogène, un alkyle en $C_1$-$C_6$,

un alcoxy en $C_1$-$C_4$, un hydroxyle, un amino, un atome d'halogène, un groupement nitro, un alkyl($C_1$-$C_6$)phényle, phényle, alkyl($C_1$-$C_6$)amino, hydroxyalkyl($C_1$-$C_6$)amino, polyhydroxyalkyl($C_2$-$C_6$)amino;

et un composant (B) constitué d'une composition contenant dans un milieu approprié pour la teinture, au moins

(i) une diamine aromatique ou un aminophénol di-, tri- ou tétrasubstitué de formule (II) suivante :

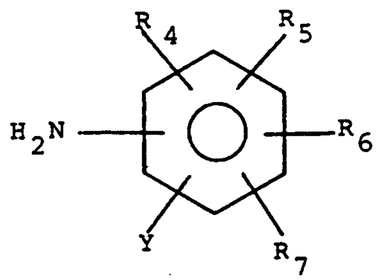

(II)

dans laquelle :

Y désigne OH ou $NR_8R_9$;

$R_8$ et $R_9$, identiques ou différents, désignent hydrogène, aminoéthyle, hydroxyéthyle ou alkyle en $C_1$-$C_4$;

$R_4$ à $R_7$, indépendamment ent l'un de l'autre, désignent un atome d'hydrogène, un alkyle en $C_1$-$C_4$, un chlore, un acétylamino, un alcoxy en $C_1$-$C_4$, ou un aryloxy;

au plus deux des groupements $R_4$ à $R_7$ désignant un atome d'hydrogène;

à l'exclusion du 2,5-diméthoxy 1,4-diaminobenzène;

ainsi que les sels cosmétiquement acceptables; ou

(ii) une bis-phénylalkylènediamine, dite base double, de formule (IV) :

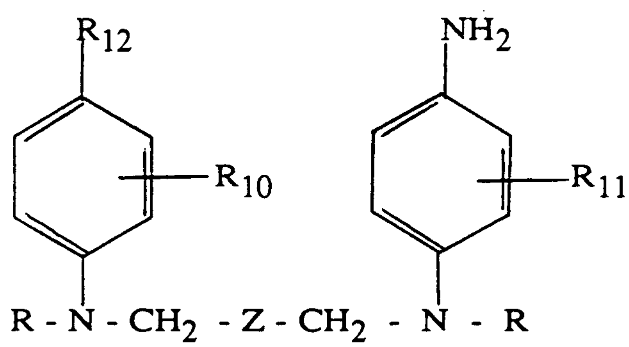

(IV)

dans laquelle :

$R_{12}$ représente un groupement hydroxyle ou $NHR_{13}$, où $R_{13}$ désigne un atome d'hydrogène ou un radical alkyle inférieur;

$R_{10}$ et $R_{11}$, identiques ou différents, représentent soit des atomes d'hydrogène, soit des atomes d'halogène, soit encore des groupements alkyle;

R représente un atome d'hydrogène, un groupe alkyle, hydroxyalkyle ou aminoalkyle, dont le reste amino peut être substitué;

Z représente un radical pris dans le groupe constitué par les radicaux suivants :

$-(CH_2)_n-$, $-(CH_2)_{n'}-O-(CH_2)_{n'}-$,

$-(CH_2)_{n'}-CHOH-(CH_2)_{n'}-$,

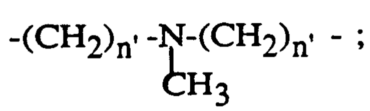

n est un nombre entier compris entre 0 et 8 n′ un nombre entier compris entre 0 et 4, ainsi que ses sels d'addition avec des acides.

**2.** Procédé selon la revendication 1, caractérisé par le fait que le composé de formule (I) est l'isatine.

**3.** Procédé selon la revendication 1 ou 2, caractérisé par le fait que le composé de formule (II) est choisi parmi :

le 2-méthoxy 3,5-diméthyl 1,4-diaminobenzène
le 2,6-diméthyl 4-bis-(β-hydroxyéthyl)amino 1-aminobenzène
le 5,6-diméthoxy 1,3-diaminobenzène
le 2,6-diméthyl 1,3-diaminobenzène
le 2,6-diméthoxy 1,3-diaminobenzène
le 2,6-diméthoxy 5-chloro 1,3-diaminobenzène
le 2,6-diméthoxy 3-β-hydroxyéthylamino 1-aminobenzène
le 2,4-diméthoxy 3-β-hydroxyéthylamino 1-aminobenzène
le 4,6-dibenzyloxy 1,3-diaminobenzène
le 3-méthyl 6-méthoxy 1,2-diaminobenzène
le 3,5-diméthyl 4-aminophénol
le 2,5-diméthyl 4-aminophénol
le 2,3,5-triméthyl 4-aminophénol
le 2,3,5,6-tétraméthyl 4-aminophénol
le 4-chloro 5-acétylamino 2-aminophénol, et
le 4,6-diphényloxy 1,3-diaminobenzène.

**4.** Procédé selon la revendication 1 ou 2, caractérisé par le fait que le composé de formule (II) est choisis parmi :

le 2,6-diméthyl 1,4-diaminobenzène
le 2,5-diméthyl 1,4-diaminobenzène
le 2,3-diméthyl 1,4-diaminobenzène
le 2-méthoxy 5-méthyl 1,4-diaminobenzène
le 2-méthoxy 5-méthyl 4-β-aminoéthylamino 1-aminobenzène
le 2-méthyl 5-chloro 4-β-aminoéthylamino 1-aminobenzène
le 4,6-diméthoxy 1,3-diaminobenzène
le 2,3-diméthyl 4-aminophénol
le 2,6-diméthyl 4-aminophénol, et
le 2,5-diméthyl 3-aminophénol.

**5.** Procédé selon la revendication 1 ou 2, caractérisé par le fait que la bis-phénylalkylènediamine de formule (IV) est choisie parmi le N,N′bis-(β-hydroxyéthyl)N,N′-bis(4′-aminophényl) 1,3-diamino 2-propanol, la N,N′bis-(β-hydroxyéthyl)N,N′-bis(4′-aminophényl)éthylène diamine, la N,N′bis-(4-aminophényl)-tétraméthylènediamine, la N,N′bis-(β-hydroxyéthyl)N,N′bis(4-aminophényl)tétraméthylène diamine, la N,N′bis(éthyl)N,N′-bis(4'-amino 3'-méthylphényl) éthylène diamine.

**6.** Procédé selon l'une quelconque des revendications 1 à 5, caractérisé par le fait que les composés de formule (I) sont présents dans le composant (A) dans des proportions comprises entre 0,01 et 5% en poids par rapport au poids du composant (A) ou au poids total de (A) + (B) et que les composés de formule (II) ou (IV) sont présents dans le composant (B) dans des proportions comprises entre 0,01 et 55% en poids par rapport au poids du composant (B) ou du poids total de (A) + (B).

**7.** Procédé selon l'une quelconque des revendications 1 à 6, caractérisé par le fait que le composant (A) et/ou le composant (B) est une composition aqueuse ou anhydre sous forme liquide plus ou moins épaissi, une composition sous forme de crème, de gel aqueux ou anhydre, d'huile ou de poudre à diluer avec un liquide au moment de l'emploi.

**8.** Procédé selon la revendication 7, caractérisé par le fait que le composant (A) et/ou le composant (B) se présente(nt) sous forme de composition(s) aqueuse(s) ayant un pH compris entre 2 et 10 et contenant un ou plusieurs adjuvants cosmétiquement acceptables, choisis parmi les agents tensio-actifs anioniques, cationiques, nonioniques ou leurs mélanges, des solvants organiques, des polymères anioniques,

non-ioniques, cationiques, amphotères ou leurs mélanges, des agents épaississants, des agents de pénétration, des agents séquestrants, des agents antioxydants, des tampons, des colorants, des parfums.

**9.** Procédé selon la revendication 7, caractérisé par le fait que le composant (A) et/ou le composant (B) se présente(nt) sous forme de composition(s) anhydre(s) contenant un ou plusieurs solvants anhydres et un ou plusieurs tensio-actifs anhydres, dans des proportions d'au moins 15% de solvant et d'au moins 20% d'agent tensio-actif.

**10.** Procédé selon la revendication 9, caractérisé par le fait que le solvant anhydre est choisi parmi les monoalcools saturés en $C_2$-$C_{20}$, les polyols, les éthers de glycol, les esters de glycol, les esters d'acide gras d'alcools inférieurs.

**11.** Procédé selon la revendication 7, caractérisé par le fait que le composant (A) et/ou le composant (B) se présente(nt) sous forme de poudre, à diluer avec un liquide au moment de l'emploi, constituée par des substances amylacées ou mucilagineuses ou par des poudres ou farines choisies parmi les silices, les argiles, les végétaux pulvérisés après extraction de leurs principes actifs par des solvants.

**12.** Procédé selon la revendication 11, caractérisé par le fait que l'on réalise un cataplasme à partir du composant (A) et/ou du composant (B) sous forme de poudre, par addition d'un liquide cosmétiquement acceptable, dans des proportions suffisantes pour obtenir une viscosité de 0,3 à 5 Pa.s.

**13.** Procédé selon l'une quelconque des revendications 1 à 12, caractérisé par le fait que l'on mélange les composants (A) et (B) juste avant emploi, que l'on applique immédiatement la composition résultante sur les fibres kératiniques, qu'on laisse agir pendant 5 à 40 minutes; les fibres kératiniques étant ensuite rincées, lavées au shampooing, rincées à nouveau, puis séchées.

**14.** Procédé selon l'une quelconque des revendications 1 à 12, caractérisé par le fait qu'il comporte l'application sur les fibres kératiniques du composant (A) suivie ou précédée de l'application sur lesdites fibres du composant (B), que l'on laisse agir chaque composant pendant 5 à 40 minutes, que l'on procède éventuellement à un rinçage à l'eau entre chaque application; les fibres kératiniques étant ensuite rincées, lavées au shampooing, rincées à nouveau, puis séchées.

**15.** Agent de teinture des fibres kératiniques et en particulier des cheveux, caractérisé par le fait qu'il comporte les composants (A) et (B) tels que définis dans les revendications 1 à 12, sous forme séparée; les composants (A) et (B) étant destinés à être, soit mélangés tout juste avant emploi, soit appliqués de façon successive sur les fibres à traiter.

**16.** Dispositif à plusieurs compartiments ou "kit de teinture", caractérisé par le fait qu'il comporte au moins deux compartiments dont un renferme le composant (A) tel que défini dans l'une quelconque des revendications 1, 2, 6 à 12, et le second renferme le composant (B) tel que défini dans l'une quelconque des revendications 1, 3 à 12.

**17.** Dispositif selon la revendication 16, caractérisé par le fait que le composant (A) et/ou le composant (B) se présente(nt) sous forme de composition anhydre et qu'il comporte un troisième compartiment contenant un milieu aqueux cosmétiquement acceptable approprié pour la teinture destiné à être mélangé avant emploi dans l'un ou les deux premiers compartiments renfermant chaque composant (A) ou (B).

## Claims

**1.** Process for dyeing keratinous fibres, in particular human hair, characterised by applying to the said fibres a component (A) consisting of a composition containing, in a suitable medium for dyeing, at least one compound of formula (I):

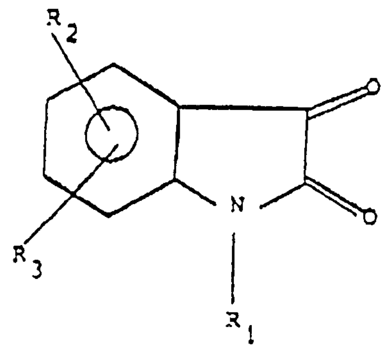

(I)

in which:

$R_1$ denotes a hydrogen atom or a $C_1$-$C_6$ alkyl, acetyl, benzoyl, phenyl or $C_1$-$C_4$ carboxyalkyl radical,

$R_2$ and $R_3$ independently of each other denote a hydrogen atom, a $C_1$-$C_6$ alkyl, a $C_1$-$C_4$ alkoxy, a hydroxyl, an amino, a halogen atom, a nitro group, an alkyl($C_1$-$C_6$)-phenyl, phenyl, $C_1$-$C_6$ alkylamino, $C_1$-$C_6$ hydroxyalkylamino or poly($C_2$-$C_6$ hydroxyalkyl)amino,

and a component (B) consisting of a composition containing, in a suitable medium for dyeing, at least

(i) an aromatic diamine or a di-, tri- or tetra-substituted aminophenol of the following formula (II):

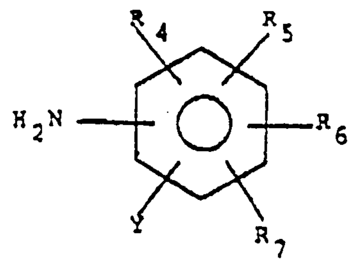

(II)

in which:

Y denotes OH or $NR_8R_9$,

$R_8$ and $R_9$, which are identical or different, denote hydrogen, aminoethyl, hydroxyethyl or $C_1$-$C_4$ alkyl,

$R_4$ to $R_7$ independently of each other denote a hydrogen atom, a $C_1$-$C_4$ alkyl, a chlorine, an acetylamino, a $C_1$-$C_4$ alkoxy or an aryloxy,

not more than two of the groups $R_4$ to $R_7$ denoting a hydrogen atom,

excluding 2,5-dimethoxy-1,4-diaminobenzene,

as well as the cosmetically acceptable salts of these compounds, or

(ii) a bisphenylalkylenediamine, called a double base, of formula (IV)

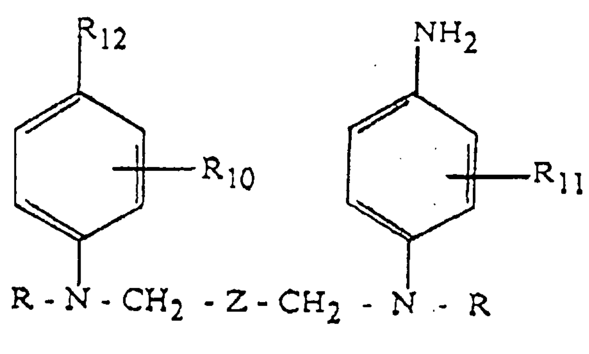

(IV)

in which:

$R_{12}$ denotes a hydroxyl or $NHR_{13}$ group, where $R_{13}$ denotes a hydrogen atom or a lower alkyl

radical,

$R_{10}$ and $R_{11}$, which are identical or different, denote either hydrogen atoms or halogen atoms or else alkyl groups,

R denotes a hydrogen atom or an alkyl, hydroxyalkyl or aminoalkyl group in which the amino residue may be substituted,

Z denotes a radical taken from the group consisting of the following radicals:

$-(CH_2)_n-$, $-(CH_2)_{n'}-O-(CH_2)_{n'}-$,

$(CH_2)_{n'}-CHOH-(CH_2)_{n'}$,

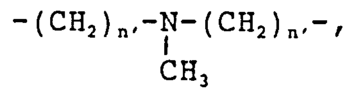

n being an integer from 0 to 8 and n' an integer between 0 and 4, and its addition salts with acids.

**2.** Process according to Claim 1, characterised in that the compound of formula (I) is isatin.

**3.** Process according to Claim 1 or 2, characterised in that the compound of formula (II) is chosen from:

2-methoxy-3,5-dimethyl-1,4-diaminobenzene
2,6-dimethyl-4-bis(β-hydroxyethyl)amino-1-aminobenzene
5,6-dimethoxy-1,3-diaminobenzene
2,6-dimethyl-1,3-diaminobenzene
2,6-dimethoxy-1,3-diaminobenzene
2,6-dimethoxy-5-chloro-1,3-diaminobenzene
2,6-dimethoxy-3-β-hydroxyethylamino-1-aminobenzene
2,4-dimethoxy-3-β-hydroxyethylamino-1-aminobenzene
4,6-dibenzyloxy-1,3-diaminobenzene
3-methyl-6-methoxy-1,2-diaminobenzene
3,5-dimethyl-4-aminophenol
2,5-dimethyl-4-aminophenol
2,3,5-trimethyl-4-aminophenol
2,3,5,6-tetramethyl-4-aminophenol
4-chloro-5-acetylamino-2-aminophenol, and
4,6-diphenyloxy-1,3-diaminobenzene.

**4.** Process according to Claim 1 or 2, characterised in that the compound of formula (II) is chosen from

2,6-dimethyl-1,4-diaminobenzene
2,5-dimethyl-1,4-diaminobenzene
2,3-dimethyl-1,4-diaminobenzene
2-methoxy-5-methyl-1,4-diaminobenzene
2-methoxy-5-methyl-4-β-aminoethylamino-1-aminobenzene
2-methyl-5-chloro-4-β-aminoethylamino-1-aminobenzene
4,6-dimethoxy-1,3-diaminobenzene
2,3-dimethyl-4-aminophenol
2,6-dimethyl-4-aminophenol, and
2,5-dimethyl-3-aminophenol.

**5.** Process according to Claim 1 or 2, characterised in that the bisphenylalkylenediamine of formula (IV) is chosen from N,N'-bis(β-hydroxyethyl)-N,N'-bis(4'-aminophenyl)-1,3-diamino-2-propanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine and N,N'-bis(ethyl)-N,N'-bis(4'-amino-3'-methylphenyl)ethylenediamine.

**6.** Process according to any one of Claims 1 to 5, characterised in that the compounds of formula (I) are present in the component (A) in proportions of between 0.01 and 5% by weight relative to the weight of the component (A) or to the total weight of (A) + (B) and in that the compounds of formula (II) or (IV) are present in the component (B) in proportions of between 0.01 and 5% by weight relative to the

weight of the component (B) or of the total weight of (A) + (B).

**7.** Process according to any one of Claims 1 to 6, characterised in that the component (A) and/or the component (B) is an aqueous or anhydrous composition in more or less thickened liquid form, a composition in the form of cream, aqueous or anhydrous gel, oil or powder to be diluted with a liquid at the time of use.

**8.** Process according to Claim 7, characterized in that the component (A) and/or the component (B) is(are) in the form of aqueous composition(s) having a pH of between 2 and 10 and containing one or more cosmetically acceptable adjuvants chosen from anionic, cationic or nonionic surface-active agents or mixtures thereof, organic solvents, anionic, nonionic, cationic or amphoteric polymers or mixtures thereof, thickening agents, penetrating agents, sequestering agents, antioxidants, buffers, dyes and perfumes.

**9.** Process according to Claim 7, characterised in that the component (A) and/or the component (B) is(are) in the form of anhydrous composition(s) containing one or more anhydrous solvents and one or more anhydrous surfactants in proportions of at least 15% of solvent and of at least 20% of surface-active agent.

**10.** Process according to Claim 9, characterised in that the anhydrous solvent is chosen from saturated $C_2$-$C_{20}$ monoalcohols, polyols, glycol ethers, glycol esters and fatty acid esters of lower alcohols.

**11.** Process according to Claim 7, characterized in that the component (A) and/or the component (B) is(are) in the form of powder to be diluted with a liquid at the time of use, consisting of starchy or mucilaginous substances or of powders or flours chosen from silicas, clays and plants pulverised after extraction of their active principles with solvents.

**12.** Process according to Claim 11, characterised in that a poultice is produced from the component (A) and/or the component (B) in powder form, by adding a cosmetically acceptable liquid in sufficient proportions to obtain a viscosity of 0.3 to 5 Pa s.

**13.** Process according to any one of Claims 1 to 12, characterised in that the components (A) and (B) are mixed just before use, that the resulting composition is immediately applied to the keratinous fibres, that it is left to act for 5 to 40 minutes, the keratinous fibres being then rinsed, shampooed, rinsed again and then dried.

**14.** Process according to any one of Claims 1 to 12, characterised in that it comprises the application of the component (A) to the keratinous fibres, followed or preceded by the application of the component (B) to the said fibres, that each component is left to act for 5 to 40 minutes, that a rinsing with water is optionally carried out between each application, the keratinous fibres being then rinsed, shampooed, rinsed again and then dried.

**15.** Agent for drying keratinous fibres and in particular hair, characterised in that it comprises the components (A) and (B) as are defined in Claims 1 to 12, in separate form, the components (A) and (B) being intended to be either mixed just before use or applied successively to the fibres to be treated.

**16.** Multicompartment device or dyeing kit characterised in that it comprises at least two compartments one of which contains the component (A) as defined in any one of Claims 1, 2, 6 to 12 and the second contains the component (B) as defined in any one of Claims 1, 3 to 12.

**17.** Device according to Claim 16, characterised in that the component (A) and/or the component (B) is(are) in the form of anhydrous composition and that it comprises a third compartment containing a cosmetically acceptable aqueous medium which is suitable for the dyeing and intended to be mixed before use in one or both first compartments containing each component (A) or (B).

## Patentansprüche

1. Verfahren zur Färbung keratinischer Fasern, insbesondere menschlicher Haare, dadurch **gekennzeichnet**, daß man auf die genannten Fasern einen Bestandteil (A) aus einer Zusammensetzung, die in einem zur Färbung geeigneten Milieu mindestens eine Verbindung der Formel (I) enthält:

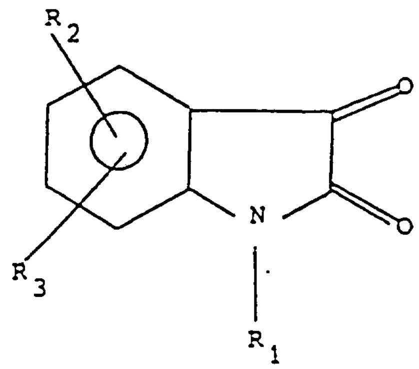

(I)

worin gilt:
$R_1$ bedeutet ein Wasserstoffatom, einen $C_{1-6}$-Alkyl-, Acetyl-, Benzoyl-, Phenyl- oder Carboxy-$C_{1-4}$-alkylrest;
$R_2$ und $R_3$ bedeuten, unabhängig voneinander, ein Wasserstoffatom, einen $C_{1-6}$-Alkyl-, $C_{1-4}$-Alkoxy-, Hydroxyl-, Aminorest, ein Halogenatom, eine Nitrogruppe, einen $C_{1-6}$-Alkylphenyl-, Phenyl-, $C_{1-6}$-Alkylamino-, Hydroxy-$C_{1-6}$-Alkylamino- und einen Polyhydroxy-$C_{2-6}$-alkylaminorest;
und einen Bestandteil (B) aus einer Zusammensetzung aufbringt, die in einem zur Färbung geeigneten Milieu mindestens
(i) ein aromatisches Diamin oder ein di-, tri- oder tetrasubstituiertes Aminophenol der folgenden Formel (II):

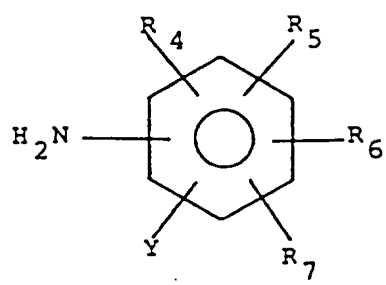

(II)

worin gilt:
Y bedeutet OH oder $NR_8R_9$;
$R_8$ und $R_9$ bedeuten, gleich oder verschieden, ein Wasserstoffatom, einen Aminoethyl-, Hydroxyethyl- oder $C_{1-4}$-Alkylrest;
$R_4$ bis $R_7$ bedeuten, unabhängig voneinander, ein Wasserstoffatom, einen $C_{1-4}$-Alkylrest, ein Chloratom, einen Acetylamino-, $C_{1-4}$-Alkoxy- oder einen Aryloxyrest;
höchstens zwei der Gruppen $R_4$ bis $R_7$ bedeuten ein Wasserstoffatom,
wobei die Verbindung 2,5-Dimethoxy-1,4-diaminobenzol ausgenommen ist,
sowie die kosmetisch verträglichen Salze dieser Verbindungen oder

(ii) ein Bisphenylalkylendiamin, Doppelbase genannt, der Formel (IV):

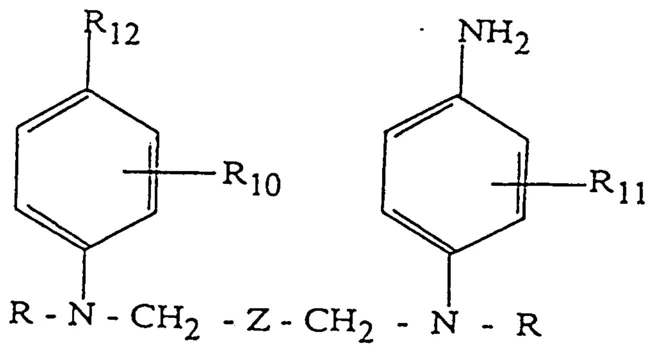

(IV)

worin gilt:

$R_{12}$ stellt eine Hydroxyl- oder $NHR_{13}$-Gruppe dar, worin $R_{13}$ ein Wasserstoffatom oder einen Niedrigalkylrest bedeutet;

$R_{10}$ und $R_{11}$ stellen, gleich oder verschieden, entweder Wasserstoffatome oder Halogenatome oder auch Alkylgruppen dar;

R stellt ein Wasserstoffatom, eine Alkyl-, Hydroxyalkyl- oder Aminoalkylgruppe dar, deren Aminorest substituiert sein kann;

Z stellt einen Rest aus der Gruppe aus den folgenden Resten dar:

$-(CH_2)_n-$, $-(CH_2)_{n'}-O-(CH_2)_{n'}-$,

$-(CH_2)_{n'}-CHOH-(CH_2)_{n'}-$,

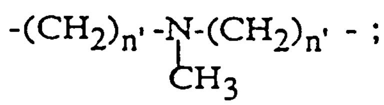

worin n eine ganze Zahl von 0 bis 8 und n' eine ganze Zahl von 0 bis 4 sind,
wobei diese Base in Form ihrer Additionssalze mit Säuren vorliegen kann,
enthält.

**2.** Verfahren gemäß Anspruch 1,
dadurch **gekennzeichnet**, daß
die Verbindung der Formel (I) Isatin ist.

**3.** Verfahren gemäß Anspruch 1 oder 2,
dadurch **gekennzeichnet**, daß
die Verbindung der Formel (II) ausgewählt ist aus:
2-Methoxy-3,5-dimethyl-1,4-diaminobenzol
2,6-Dimethyl-4-bis($\beta$-hydroxyethyl)amino-1-aminobenzol
5,6-Dimethoxy-1,3-diaminobenzol
2,6-Dimethyl-1,3-diaminobenzol
2,6-Dimethoxy-1,3-diaminobenzol
2,6-Dimethoxy-5-chlor-1,3-diaminobenzol
2,6-Dimethoxy-3-$\beta$-hydroxyethylamino-1-aminobenzol
2,4-Dimethoxy-3-$\beta$-hydroxyethylamino-1-aminobenzol
4,6-Dibenzyloxy-1,3-diaminobenzol
3-Methyl-6-methoxy-1,2-diaminobenzol
3,5-Dimethyl-4-aminophenol
2,5-Dimethyl-4-aminophenol
2,3,5-Trimethyl-4-aminophenol
2,3,5,6-Tetramethyl-4-aminophenol
4-Chlor-5-acetylamino-2-aminophenol und

4,6-Diphenyloxy-1,3-diaminobenzol

**4.** Verfahren gemäß Anspruch 1 oder 2,
dadurch **gekennzeichnet**, daß
die Verbindung der Formel (II) ausgewählt ist aus:
2,6-Dimethyl-1,4-diaminobenzol
2,5-Dimethyl-1,4-diaminobenzol
2,3-Dimethyl-1,4-diaminobenzol
2-Methoxy-5-methyl-1,4-diaminobenzol
2-Methoxy-5-methyl-4-β-aminoethylamino-1-aminobenzol
2-Methyl-5-chlor-4-β-aminoethylamino-1-aminobenzol
4,6-Dimethoxy-1,3-diaminobenzol
2,3-Dimethyl-4-aminophenol
2,6-Dimethyl-4-aminophenol und
2,5-Dimethyl-3-aminophenol.

**5.** Verfahren gemäß Anspruch 1 oder 2,
dadurch **gekennzeichnet**, daß
das Bisphenylalkylendiamin der Formel (IV) aus N,N'-Bis(β-hydroxyethyl)-N,N'-bis(4'-aminophenyl)-1,3-diaminopropan-2-ol, N,N'-Bis(β-hydroxyethyl)-N,N'-bis(4'-aminophenyl)ethylendiamin, N,N'-Bis(4-aminophenyl)tetramethylendiamin, N,N'-Bis(β-hydroxyethyl)-N,N'-bis(4-aminophenyl)tetramethylendiamin, N,N'-bis(ethyl)-N,N'-bis(4'-amino-3'-methylphenyl)ethylendiamin ausgewählt ist.

**6.** Verfahren gemäß jedem der Ansprüche 1 bis 5,
dadurch **gekennzeichnet**, daß
die Verbindungen der Formel (I) im Bestandteil (A) in Mengenanteilen von 0,01 bis 5 Gew.%, bezogen auf das Gewicht des Bestandteils (A) oder auf das Gesamtgewicht von (A) + (B), und daß die Verbindungen der Formel (II) oder (IV) im Bestandteil (B) in Mengenanteilen von 0,01 bis 5 Gew.%, bezogen auf das Gewicht des Bestandteils (B) oder auf das Gesamtgewicht von (A) + (B), vorhanden sind.

**7.** Verfahren gemäß jedem der Ansprüche 1 bis 6,
dadurch **gekennzeichnet**, daß
der Bestandteil (A) und/oder der Bestandteil (B) eine wässrige oder wasserfreie Zusammensetzung in flüssiger, mehr oder weniger verdickter Form oder eine Zusammensetzung in Form einer Creme, eines wässrigen wasserfreien Gels, eines Öls oder eines Pulvers zur zum Zeitpunkt der Anwendung mit einer Flüssigkeit durchzuführenden Verdünnung sind.

**8.** Verfahren gemäß Anspruch 7,
dadurch **gekennzeichnet**, daß
der Bestandteil (A) und/oder der Bestandteil (B) in Form einer wässrigen Zusammensetzung mit einem pH-Wert von 2 bis 10 vorliegen und einen oder mehrere kosmetisch geeignete Hilfsstoffe enthalten, ausgewählt aus anionischen, kationischen, nicht-ionischen oder amphoteren oberflächenaktiven Mitteln oder aus deren Mischungen, aus organischen Lösungsmitteln, anionischen, nicht-ionischen, kationischen oder amphoteren Polymeren oder aus deren Mischungen, aus Verdickungsmitteln, Eindringmitteln, Sequestriermitteln, Antioxidantien, Puffern, Färbemitteln, Parfüm-Produkten.

**9.** Verfahren gemäß Anspruch 7,
dadurch **gekennzeichnet**, daß
der Bestandteil (A) und/oder der Bestandteil (B) in Form einer wasserfreien Zusammensetzung vorliegt, welche ein oder mehrere wasserfreie Lösungsmittel und ein oder mehrere wasserfreie oberflächenaktive Mittel in Mengenanteilen von mindestens 15% Lösungsmittel und mindestens 20% oberflächenaktives Mittel enthält.

**10.** Verfahren gemäß Anspruch 9,
dadurch **gekennzeichnet**, daß
das wasserfreie Lösungsmittel aus gesättigten $C_{2-20}$-Monoalkoholen, Polyolen, Glycolethern, Glycolestern, Fettsäureestern von Niedrigalkoholen ausgewählt ist.

**11.** Verfahren gemäß Anspruch 7,
dadurch **gekennzeichnet**, daß
der Bestandteil (A) und/oder der Bestandteil (B) in Form eines Pulvers zur zum Zeitpunkt der Anwendung mit einer Flüssigkeit durchzuführenden Verdünnung vorliegen, wobei das Pulver aus stärkeartigen oder schleimartigen Substanzen oder aus Pulver- oder Mehl-Produkten zusammengesetzt ist, ausgewählt aus Kieselgelen, Tonen, pflanzlichen Produkten, die nach Lösungsmittelextraktion ihrer Wirkstoffe pulverisiert sind.

**12.** Verfahren gemäß Anspruch 11,
dadurch **gekennzeichnet**, daß
man ein Kataplasma aus den in Form eines Pulvers vorliegenden Bestandteilen (A) und/oder (B) zubereitet, und zwar durch Zugabe einer kosmetisch geeigneten Flüssigkeit in Mengenanteilen, die ausreichen, um eine Viskosität von 0,3 bis 5 Pa x s zu erhalten.

**13.** Verfahren gemäß jedem der Ansprüche 1 bis 12,
dadurch **gekennzeichnet**, daß
man die Bestandteile (A) und (B) kurz vor Anwendung vermischt, die sich ergebende Zusammensetzung unmittelbar auf die keratinischen Fasern aufbringt, das Ganze 5 bis 40 Minuten lang einwirken läßt, worauf die keratinischen Fasern gespült, schamponiert, erneut gespült und dann getrocknet werden.

**14.** Verfahren gemäß jedem der Ansprüche 1 bis 12,
dadurch **gekennzeichnet**, daß
man auf die keratinischen Fasern den Bestandteil (A) vor oder nach der entsprechenden Aufbringung des Bestandteils (B) appliziert und jeden Bestandteil 5 bis 40 Minuten lang einwirken läßt, wobei man gegebenenfalls zwischen jeder Aufbringung mit Wasser spült, worauf die keratinischen Fasern gespült, schamponiert, erneut gespült und dann getrocknet werden.

**15.** Mittel zur Färbung keratinischer Fasern und insbesondere der Haare,
dadurch **gekennzeichnet**, daß
es die in den Ansprüchen 1 bis 12 definierten Bestandteile (A) und (B) in getrennter Form umfaßt, wobei die Bestandteile (A) und (B) entweder kurz vor Anwendung vermischt oder nacheinander auf die zu behandelnden Fasern aufgebracht werden sollen.

**16.** Vorrichtung aus mehreren Teilstücken oder "Kit zur Färbung",
dadurch **gekennzeichnet**, daß
sie mindestens zwei Teilstücke umfassen, von denen das eine den in jedem der Ansprüche 1, 2 und 6 bis 12 definierten Bestandteil (A) und das zweite den in jedem der Ansprüche 1 und 3 bis 12 definierten Bestandteil (B) einschließen.

**17.** Vorrichtung gemäß Anspruch 16,
dadurch **gekennzeichnet**, daß
der Bestandteil (A) und/oder der Bestandteil (B) in Form einer wasserfreien Zusammensetzung vorliegen, und daß sie ein drittes Teilstück umfaßt, das ein wässriges, kosmetisch verträgliches, zur Färbung geeignetes Milieu enthält, das vor Anwendung mit dem einen oder beiden Teilen vermischt werden soll, die jeden Bestandteil (A) oder (B) einschließen.